# FASCICULE DE BREVET EUROPEEN

(11) **EP 1 140 861 B1**
(45) Date de publication et mention de la délivrance du brevet: **02.07.2003**
(21) Numéro de dépôt: 00900538.0
(22) Date de dépôt: 07.01.2000
(51) Int. Cl.: C07D 241/12, A61K 31/495

(54) **NOUVEAUX DERIVES DE POLYHYDROXYPYRAZINES, LEUR PREPARATION ET LES COMPOSITIONS PHARMACEUTIQUES QUI LES CONTIENNENT**
POLYHYDROXYPYRAZINEDERIVATE, DEREN HERSTELLUNG UND DIESE ENTHALTENDE PHARMAZEUTISCHE ZUSAMMENSTELLUNGEN
NOVEL POLYHYDROXYPYRAZINE DERIVATIVES, PREPARATION AND PHARMACEUTICAL COMPOSITIONS CONTAINING SAME

(30) Priorité: 11.01.1999 FR 9900186
(43) Date de publication de la demande: 10.10.2001
(73) Titulaire: Aventis Pharma S.A., 92160 Antony (FR)
(72) Inventeur: BOUCHARD, Hervé, F-94320 Thiais (FR); COMMERCON, Alain, F-94400 Vitry-sur-Seine (FR)
(74) Mandataire: Rousseau, Pierrick Edouard
(86) Numéro de dépôt international: FR0000026
(87) Numéro de publication internationale: WO00042027

(56) Documents cités:
- WO-A-97/28813
- FR-A- 2 766 185
- CHEMICAL ABSTRACTS, vol. 78, no. 17, 1973 Columbus, Ohio, US; abstract no. 111631m, M. TEGLIA ET AL.: "REACTION OF ACYLATED KETOSES WITH AMMONIA." page 516; colonne 1; XP002112796 & CARBOHYDR. RESEARCH, vol. 26, no. 2, 1973, pages 377-384,
- CHEMICAL ABSTRACTS, vol. 81, no. 6, 1974 Columbus, Ohio, US; abstract no. 116272s, TSUCHIDA, HIRONOBU ET AL.: "FORMATION OF DEOXYFRUCTOSASINE" page 185; colonne 2; XP002112797 & AGRICULTURAL AND BIOLOGICAL CHEMISTRY., vol. 37, no. 11, 1973, pages 2571-2578, JAPAN SOC. FOR BIOSCIENCE, BIOTECHNOLOGY AND AGROCHEM. TOKYO., JP ISSN: 0002-1369

## Description

La présente invention concerne les nouveaux produits de formule générale (I), 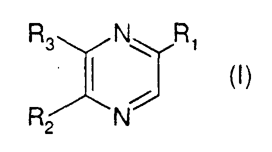 ainsi que leurs sels avec un acide minéral ou organique, leur préparation, les compositions pharmaceutiques qui les contiennent, et leur utilisation en tant qu'agent antidiabétique.

Des produits présentant des propriétés hypoglycémiantes, de formule générale : 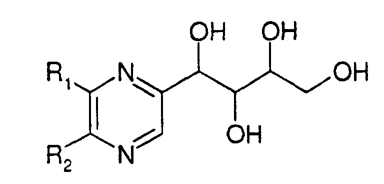 dans laquelle soit R₁ représente un atome d'hydrogène et R₂ représente une chaîne de formule:

-CH₂-CHOH-CHOH-CH₂OH (A)

-CHOH-CHOH-CHOH-CH₂OH (B)

soit R₁ représente une chaîne de formule (A) et R₂ représente un atome d'hydrogène, ont été décrits dans la demande WO 97/28813.

Cependant, rien ne laissait prévoir dans l'art antérieur que, du fait de leur modifications structurales par rapport à ces produits, les produits de formule générale (I) selon l'invention présenteraient des propriétés considérablement améliorées, tant en terme d'activité anti-glycémiante que en terme de biodisponibilité et/ou toxicité.

La présente invention conceme les produits de formule générale (I), 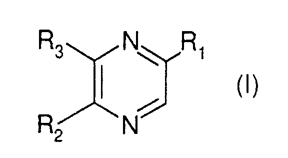 dans laquelle :
R₁ représente les formes stéréoisomères de la chaîne

-(CHOH)₃-CH₂-O-COR (II)

et
soit R₂ représente un atome d'hydrogène et R₃ représente les formes stéréoisomères de la chaîne

-CH₂-(CHOH)₂-CH₂-O-COR (III)

soit R₂ représente les formes stéréoisomères des chaînes

-(CHOH)₃-CH₂-O-COR (II)

ou

-CH₂-(CHOH)₂-CH₂-O-COR (III)

et R₃ représente un atome d'hydrogène
et
R représente un radical -(Alk)ᵢ-(Cycloalk),
pour lequel :
Alk désigne un radical alkyle,
Cycloalk désigne un radical cyloalkyle,
i est égal à 0 ou 1;
ainsi que
leurs formes stéréoisomères, et leurs sels avec un acide minéral ou organique.

On entend par :
- alkyle : un radical hydrocarboné saturé en chaîne droite ou ramifiée contenant 1 à 6 atomes de carbone tels que méthyle, éthyle, propyle, isopropyle, butyle, sec.butyle, tert.butyle, pentyle ou hexyle,
- cycloalkyle : un radical hydrocarboné cyclique saturé, contenant 5 ou 6 atomes de carbone tels que cyclopentyle, cyclohexyle.

La présente invention concerne donc les produits de formules générales 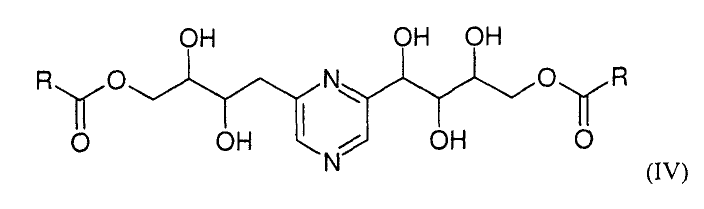 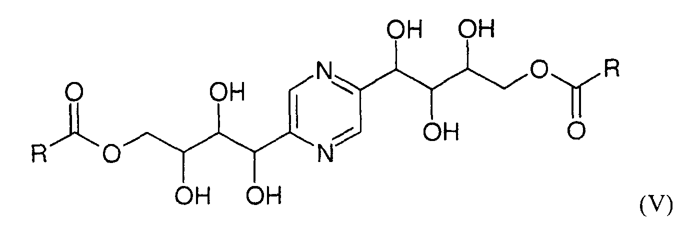 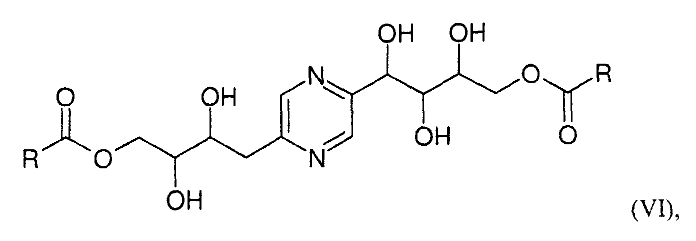 dans lesquelles
R représente un radical -(Alk)ᵢ-(Cycloalk),
pour lequel :
Alk désigne un radical alkyle,
Cycloalk désigne un radical cyloalkyle,
i est égal à 0 ou 1;
ainsi que leurs formes stéréoisomères, ou les sels de tels produits avec un acide organique ou minéral.

Selon la présente invention, on préfère les produits ayant les formules générales suivantes : 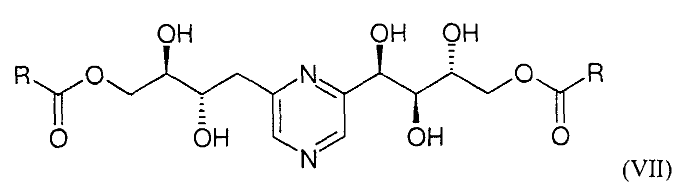 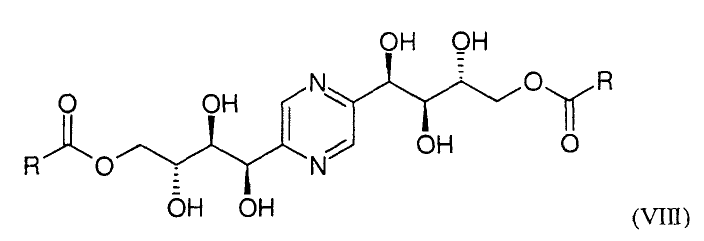 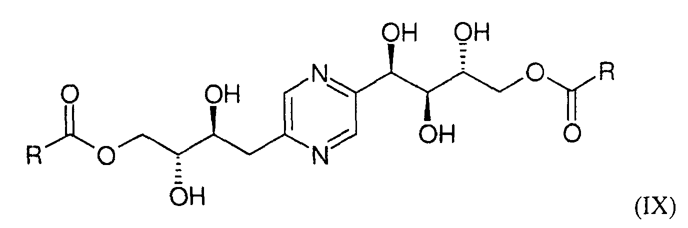 dans lesquelles
R représente un radical
-(Alk)ᵢ-(Cycloalk),
pour lequel :
Alk désigne un radical alkyle,
Cycloalk désigne un radical cyloalkyle,
i est égal à 0 ou 1;
ainsi que les sels de tels produits avec un acide organique ou minéral.

De façon encore plus préférentielle, la présente invention concerne les produits de formule générale (IX) 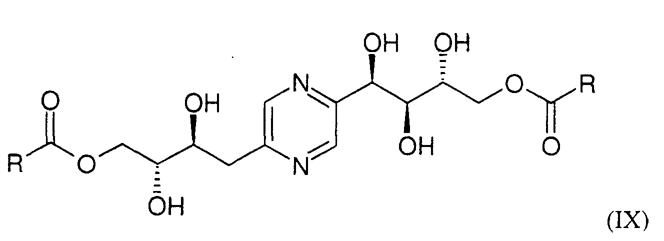 dans laquelle :
R représente un radical
-(Alk)ᵢ-(Cycloalk),
pour lequel :
Alk désigne un radical alkyle,
Cycloalk désigne un radical cyloalkyle,
i est égal à 0 ou 1;
ainsi que les sels de tels produits avec un acide organique ou minéral.

Selon un aspect encore plus avantageux, la présente invention concerne les produits de formule générale (IX) dans laquelle :
R représente un radical -(Alk)ᵢ-(Cycloalk),
pour lequel :
Alk désigne le radical méthyle,
Cycloalk désigne un radical cyclohexyle,
i est égal à 0 ou 1;
ainsi que leurs sels avec un acide minéral ou organique.

Très avantageusement, les produits selon la présente invention peuvent être choisis individuellement parmi :
4, 4'-O, O-dicyclohexyloyl-2-[(1R, 2S, 3R) (1, 2, 3, 4-tétrahydroxylbutyl)] 5-[(2'S, 3'R) (2', 3', 4'-trihydroxybutyl)] pyrazine
4, 4'-O, O-di(cyclohexyl-acétyl)-2-[(1R, 2S, 3R) (1, 2, 3, 4-tétrahydroxylbutyl)] 5-[(2'S, 3'R) (2', 3', 4'-trihydroxybutyl)] pyrazine
ainsi que leurs sels avec un acide minéral ou organique.

En particulier :
4, 4'-O, O-dicyclohexyloyl-2-[(1R, 2S, 3R) (1, 2, 3, 4-tétrahydroxylbutyl)] 5-[(2'S, 3'R) (2', 3', 4'-trihydroxybutyl)] pyrazine
ainsi que ses sels avec un acide minéral ou organique.

Selon la présente invention, les produits de formule générale (I) dans laquelle R est défini comme précédemment peuvent être obtenus à partir des produits de formule générale: 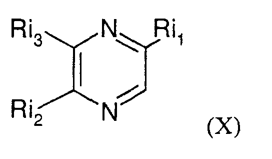 dans laquelle :
Ri₁ représente les formes stéréoisomères de la chaîne

   -(CHOH)₃-CH₂OH (XI)

   et
soit Ri₂ représente un atome d'hydrogène et Ri₃ représente les formes stéréoisomères de la chaîne

   -CH₂-(CHOH)₂-CH₂OH (XII)
soit Ri₂ représente les formes stéréoisomères des chaînes

   -(CHOH)₃-CH₂OH (XI)

   ou

   -CH₂-(CHOH)₂-CH₂OH (XII)
et Ri₃ représente un atome d'hydrogène,
par action d'un halogénure d'acyle correspondant, de formule R-COX, dans laquelle R est défini comme précédemment et X représente un atome d'halogène, tel que le chlore.
Cette réaction s'effectue en présence d'une base organique ou minérale, de préférence la pyridine, à des températures comprises entre 0 et 40°C.

L'halogénure d'acyle de formule R-COX, dans laquelle R est défini comme précédemment et X représente un atome d'halogène, tel que le chlore, peut être disponible commercialement ou éventuellement préparé à partir de l'acide correspondant R-COOH, selon les méthodes habituelles; notamment le chlorure d'acyle peut être préparé à partir de l'acide correspondant, par action du chlorure d'oxalyle, dans un solvant tel que le dichlorométhane, le N,N-diméthylformamide, ou un mélange de ces deux solvants.
Avantageusement, l'halogénure d'acyle peut être préparé in situ.

Les produits de formule générale (X) peuvent être préparés de la façon suivante :

Les formes stéréoisomères des produits de formule générale (X) sont obtenus à partir des formes stéréoisomères des réactifs ci-après utilisés par le procédé de préparation selon l'invention.

Les stéréoisomères des produits de formule (X) pour lesquels Ri₁ représente les formes stéréoisomères de la chaîne -(CHOH)₃-CH₂OH (XI), Ri₂ représente un atome d'hydrogène et Ri₃ représente les formes stéréoisomères de la chaîne -CH₂-(CHOH)₂-CH₂OH (XII) peuvent être obtenus par action du formiate d'ammonium sur un aldose, ou un mélange de 2 aldoses, de série dextrogyre ou lévogyre de formule générale :

CHO-CHOH-Ri₁ (XIII)

dans laquelle Ri₁ a la même signification que dans la formule (X).

Cette réaction peut être effectuée préférentiellement à une température comprise entre 15°C et 100°C, de préférence en milieu aqueux.

Les aldoses sont commercialisés ou peuvent être obtenus à partir :
a) d'aldoses commercialement disponibles :
   - par des réactions d'épimérisation par application ou adaptation des méthodes décrites dans Adv. Carbohydr. Chem., 13, 63, (1958) notamment en milieu basique au moyen d'une solution aqueuse diluée de soude (0,03 à 0,05%), à une température comprise entre 20 et 40°C,
   - par des réactions d'allongement de chaîne par application ou adaptation des méthodes décrites dans «The Carbohydrates», éditeurs: W. Pigman et D. Horion, Academic Press, New-York, Volume IA, 133 (1972) et notamment en formant la cyanhydrine de l'aldose de départ (par exemple par action du cyanure de sodium en solution aqueuse, à une température comprise entre 10 et 30°C et en présence de soude, à un pH voisin de 9) puis hydrolyse de la fonction nitrile ainsi formée en acide correspondant par application ou adaptation des méthodes décrites dans Organic Synthesis volume I page 436 et volume III page 85 (par exemple à l'aide d'acide chlorhydrique ou d'acide sulfurique concentré, en solution aqueuse, à une température comprise entre 20°C et la température de reflux du milieu réactionnel), puis réduction de la fonction acide carboxylique en aldéhyde correspondant par application ou adaptation des méthodes décrites dans J. Am. Chem. Soc. 71, 122 (1949) notamment à l'aide d'un borohydrure d'un métal alcalin (le borohydrure de sodium par exemple), en solution aqueuse à une température comprise entre 20°C et la température d'ébullition du milieu réactionnel,
   - par des réactions de raccourcissement de chaînes par application ou adaptation des méthodes décrites dans «The Carbohydrates», éditeurs: W. Pigman et D. Horton, Academic Press, New-York, Volume IB, 1980, page 929 ou Chem. Ber., 83, 559 (1950) et notamment en transformant la fonction aldéhyde de l'aldose en hydroxylamine correspondant par application ou adaptation des méthodes décrites dans Organic Synthesis volume II page 314 (par exemple à l'aide de chlorhydrate d'hydroxylamine, en solution aqueuse et en présence d'une base telle que le carbonate de sodium à une température comprise entre 20 et 50°C), puis action du 3,4-dinitrofluorobenzène en présence de dioxyde de carbone et d'une base telle le d'hydrogénocarbonate de sodium en solution aqueuse et d'un alcool aliphatique (alcool isopropylique par exemple), à une température comprise entre 50 et 80°C,
b) d'alcools allyliques correspondants par application ou adaptation des méthodes décrites dans Science, 220, 949 (1983) et notamment à l'aide d'hydroperoxyde de terbutyle en présence d'un complexe de titane (IV) tel que le complexe isopropylate de titane (IV) et tartrate de dialkyle optiquement pur (le tartrate de diéthyle par exemple), suivi de l'action successive de thiophénolate de sodium, d'acide parachloroperbenzoïque dans l'anhydride acétique et d'hydrure de diisopropylaluminium

Les stéréoisomères du sucre de formule (XIII) peuvent être ceux des aldoses à 6 atomes de carbone; ceux utilisés de façon préférentielle sont le D-glucose, le D-gulose, D-mannose, le D-galactose, le D-allose, le D-altrose, le D-idose, le D-talose, le L-glucose, le L-mannose, le L-galactose, le L-allose, le L-altrose, le L-idose, le L-talose, le L-gulose.

Les stéréoisomères des produits de formule (X) pour lesquels Ri₁ représente les formes stéréoisomères de la chaîne -(CHOH)₃-CH₂OH (XI), Ri₂ représente les formes stéréoisomères des chaînes -(CHOH)₃-CH₂OH (XI) et Ri₃ représente un atome d'hydrogène, peuvent être obtenus par traitement en milieu basique d'un amino-aldose, ou d'un mélange de 2 amino-aldoses de formule générale :

CHO-CH(NH₂)-Ri₁ (XIV)

éventuellement sous forme de sel d'addition, tel que le chlorhydrate, dans laquelle Ri₁ a la même signification que dans la formule générale (X).

De préférence, on opère à une température voisine de 20°C et on utilise préférentiellement une solution d'ammoniaque et plus particulièrement une solution à 28%.

Les amino-aldoses de formule (XIV) sont commercialisés ou peuvent être préparés par application ou adaptation des méthodes décrites par exemple dans :
(a) Methods Carbohydr. Chem., 7, 29 (1976) qui consistent à transformer la fonction aldéhyde de l'aldose correspondant en un groupement nitroéthylénique à l'aide du nitrométhane en milieu basique (éthylate de sodium par exemple) puis à traiter le produit obtenu successivement par une solution saturée d'ammoniaque, à une température comprise entre 20°C et 30°C, par du Ba(OH)₂ en solution aqueuse, à une température comprise entre 20°C et 30°C et enfin de l'acide sulfurique dilué (10 à 15%), à une température comprise entre 20°C et 30°C,
(b) «The Amino Sugar» , éditeur: R. W. Jeanloz, Academic Press, New-York, 1969, page 1 ou «The Carbohydrates», éditeurs: W. Pigman et D. Horton, Academic Press, New-York, Volume IB, 1980, page 664 qui consistent à transformer la fonction aldéhyde de l'aldose correspondant en un groupement imino à partir d'une amine primaire aromatique (aniline par exemple), de faire ensuite successivement réagir l'acide cyanhydrique, à une température comprise entre 0°C et 20°C et de l'hydrogène en présence de palladium dans un solvant tel qu'un éther (tétrahydrofuranne par exemple) ou un alcool aliphatique (l'éthanol ou le méthanol par exemple), à une température comprise entre 20°C et 50°C.

Les stéréoisomères de l'amino-aldose de formule (XIV) peuvent être ceux des amino-aldose à 6 atomes de carbone, tels que la D-glucosamine, la D-galactosamine, la L-glucosamine, la L-galactosamine; ceux utilisé de façon préférentielle sont la D-glucosamine, la D-galactosamine et notamment la D-glucosamine, éventuellement sous forme de sel d'addition tel que le chlorhydrate.

Les stéréoisomères des produits de formule (X) pour lesquels Ri₁ représente les formes stéréoisomères de la chaîne -(CHOH)₃-CH₂OH (XI), Ri₂ représente les formes stéréoisomères des chaînes -CH₂-(CHOH)₂-CH₂OH (XII)et Ri₃ représente un atome d'hydrogène peuvent être obtenus
soit à partir d' un amino-aldose, ou d'un mélange de 2 aminoaldoses, de formule générale :

CHO-CH(NH₂)-Ri₁ (XIV)

dans laquelle Ri₁ a la même signification que dans la formule générale (I) en milieu acide et plus particulièrement en milieu acide acétique et en opérant de préférence à une température comprise entre 15°C et 100°C.
soit à partir d'un cétose, ou d'un mélange de 2 cétoses de formule générale :

HOCH₂-CO-Ri₁ (XV)

dans laquelle Ri₁ a la même signification que dans la formule générale (X), par action du formiate d'ammonium et en opérant préférentiellement à une température comprise entre 15°C et 100°C, et de préférence en milieu aqueux.

Les cétoses de formule (XV) sont commercialisés ou peuvent être préparés par application ou adaptation des méthodes décrites par exemple dans :
a) Adv. Carbohydr. Chem., 13, 63 (1958) qui consistent à faire réagir l'aldose correspondant soit avec une base telle l'hydroxyde de calcium, la soude, la pyridine, la quinoléine, soit avec un acide tel que l'acide sulfurique en solution aqueuse ou en phase pure, à une température comprise entre 20 et 50°C
b) Tetrahedron Asymmetry, 7(8), 2185, (1996), J. Am. Chem; Soc., 118(33), 7653 (1996), J. Org. Chem., 60(13), 4294(1995), Tetrahedron Lett., 33(36), 5157(1992), J. Am. Chem. Soc., 113(17), 6678 (1991), Angew. Chem., 100(5), 737, (1988), J. Org. Chem., 57, 5899 (1992) qui consistent par exemple à condenser soit l'hydroxypyruvaldéhyde, la 1,3-dihydroxyacétone, le 1,3-dihydroxyacétone monophosphate ou l'acide hydroxypyruvique sur un 2-hydroxy-acétaldéhyde substitué en position 2, éventuellement optiquement pur, en présence éventuellement d'une enzyme telle qu'une transcétolase. Cette réaction s'éffectue généralement en solution aqueuse, à une température comprise entre 20 et 50°C, éventuellement en présence d'une base (la soude par exemple), de chlorure de barium, de chlorure de magnésium ou de chlorure de zinc. Les dérivés possédant un groupement 2-hydroxy-acétaldéhyde sont commercialisés ou peuvent être préparés à partir d'aldoses par application ou adaptation des méthodes décrites dans P. Collins, R. Ferrier, Monosaccharides. Their Chemistry and their roles in Natural Products, éditeur J. Wiley (1995), M. Bols, Carbohydrate Building Bloks, éditeur J. Wiley (1996).

Les amino-aldoses de formule (XIV) peuvent être ceux des amino-aldose à 6 atomes de carbone, tels que la D-glucosamine, la D-galactosamine, la L-glucosamine, la L-galactosamine; ceux utilisé de façon préférentielle sont la D-glucosamine. la D-galactosamine et notamment la D-glucosamine, éventuellement sous forme de sel d'addition tel que le chlorhydrate.

Les stéréoisomères des produits de formule (XV) peuvent être ceux des cétoses à 6 atomes de carbone; ceux utilisés préférentiellement sont le D-psicose, le D-fructose, le D-sorbose, le D-tagatose, le L-psicose, le L-fructose, le L-sorbose, le L-tagatose.

Les mélanges réactionnels obtenus par les divers procédés décrits précédemment sont traités suivant des méthodes classiques physiques (évaporation, extraction, distillation, chromatographie, cristallisation par exemple) ou chimiques ( formation de sels par exemple).

Les produits de formule (I) peuvent être éventuellement transformés en sels d'addition avec un acide minéral ou organique par action d'un tel acide au sein d'un solvant tel qu'un alcool, une cétone, un éther ou un solvant chloré. Ces sels font également partie de l'invention.

Comme exemple de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-b-oxynaphtoate, chlorhydrate, sulfate, nitrate et phosphate.

Les produits de formule (I) présentent des propriétés pharmacologiques intéressantes. Ce sont des hypoglycémiants.

L'activité hypoglycémiante des produits de formule (I) a été déterminée sur la réponse hyperglycémique à l'administration de glucose par la voie orale chez la souris normoglycémique, selon le protocole décrit à l'exemple 3.

Les produits de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type II (NID diabète), du diabète de l'obèse, du diabète de la cinquantaine, du diabète métapléthorique, du diabète du sujet âgé et du diabète léger. Ils peuvent être utilisés en complément de l'insulinothérapie dans le diabète insulino-dépendant où ils permettent de diminuer progressivement la dose d'insuline, le diabète instable, le diabète insulino-résistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt artérioveineux, dilatation veineuse, hémorragies ponctiformes et maculaires, exsudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies. neuropathies autonomes, amyotrophies diabétiques), les manifestations du pied diabétique (ulcères des extrémités inférieures et du pied), la néphropathie diabétique dans ses deux formes diffuse et nodulaire, l'athéromatose (élévation des HDL lipoproteines favorisant l'élimination du cholestérol à partir des plaques d'athérome, baisse des LDL lipoproteines, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéines A et B), de la cataracte, de l'hypertension artérielle et ses conséquences.

Les médicaments selon l'invention sont constitués par un produit selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

L'exemple 4 suivant illustre des compositions selon l'invention.

L'invention concerne également l'utilisation des produits de formule générale (I) pour la préparation de compositions pharmaceutiques utiles pour le traitement et/ou la prévention du diabète et les complications du diabète.

Les exemples suivants illustrent plus particulièrement et à titre non limitatif le procédé de préparation utilisé selon l'invention. Il fait partie des connaissances générales de l'homme du métier d'appliquer ou adapter ces méthodes afin de mettre en oeuvre l'invention.

### Exemple 1

### 4, 4'-O,O-dicyclohexyloyl-2-[(1R, 2S, 3R) (1, 2, 3, 4-tétrahydroxylbutyl)] 5-[(2'S, 3'R) (2', 3', 4'-trihydroxybutyl)] pyrazine

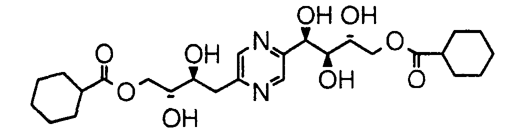

A 300 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 7,5 cm³ de pyridine séchée sur tamis moléculaire 4Å sont additionnés, goutte à goutte, à une température voisine de 20°C sous atmosphère d'argon, 0,328 cm³ de chlorure de cyclohexanoyle. La suspension blanche réactionnelle est agitée 15 heures à une température voisine de 20°C. Le milieu réactionnel est dilué avec 30 cm³ d'acétate d'éthyle et 30 cm³ d'eau distillée. Après décantation, la phase organique est lavée avec 20 cm³ d'une solution aqueuse saturée en chlorure de sodium, séchée sur sulfate de magnésium, filtrée sur verre fritté puis concentrée à sec sous pression réduite (0,2 kPa) à une température voisine de 40°C. On obtient une huile jaune que l'on purifie par chromatographie préparative sur 4 plaques de gel de silice 60F254 Merck (épaisseur = 0,5 mm, 20x20 cm) en éluant par un mélange dichlorométhane-méthanol (90-10 en volumes). Les fractions ne contenant que les produits cherchés sont extraites par un mélange dichlorométhane-méthanol (80-20 en volumes), filtrées sur verre fritté puis concentrées à sec sous pression réduite (0,5 kPa) à une température voisine de 40°C. On obtient ainsi 23,3 mg de 4, 4'-O, O-dicyclohexyloyl-2-[(1R, 2S, 3R) (1, 2, 3, 4-tétrahydroxylbutyl)] 5-[(2'S, 3'R) (2', 3', 4'-trihydroxybutyl)] pyrazine sous forme d'un solide blanc.

Le produit obtenu possède les caractéristiques suivantes :
Spectre de R.M.N. ¹H (400 MHz, (CD₃)₂SO d6, δ en ppm) : de 1,10 à 1,95 (mt, 20H en totalité : CH₂ cyclohexyle) ; 2,34 (mt, 2H : OCOCH) ; 2,76 et 3,12 (2 dd, respectivement J = 14 et 10 Hz et J = 14 et 3 Hz, 1H chacun : CH₂ 5α) ; de 3,50 à 3,65 (mt, 1H : CH 5γ) ; 3,60 (t large, J = 8,5 Hz, 1H : CH 2β) ; 3,78 (mt, 1H : CH 5β) ; 3,86 (mt, 1H : CH 2γ) ; de 3,95 à 4,05 (mt, 2H : 1H du CH₂O 5δ et 1H du CH₂O 2δ) ; 4,24 (dd, J = 11 et 3 Hz , 1H : l'autre H du CH₂O 5δ) ; 4,30 (dd, J = 12 et 2,5 Hz , 1H : l'autre H du CH₂O 2δ) ; 4,63 (d, J = 8,5 Hz, 1H : OH en 2β) ; 4,86 (d, J = 7 Hz, 1H : OH en 5β) ; 4,97 (d large, J = 6.5 Hz, 1H : CH 2α) ; 5,05 (d, J = 6 Hz, 1H : OH en 2γ) ; 5,11 (d, J = 6 Hz, 1H: OH en 5γ) ; 5,40 (d, J = 6,5 Hz, 1H : OH en 2α) ; 8,43 (s large, 1H : =CH en 6) ; 8,67 (s large, 1H : =CH en 3).
La 2-[(1R, 2S, 3R) (1, 2, 3, 4-tétrahydroxybutyl)] 5-[(2'S, 3'R) (2', 3', 4'-trihydroxybutyl)] pyrazine peut être préparée à partir de D-glucosamine, en présence d'acide acétique, ou encore par application ou adaptation des méthodes décrites dans Advances in Carbohydrate Chemistry and Biochemistry, Acadelic Press, New York, vol.25, 1970, 311-349.

### Exemple 2

### 4, 4'-O, O-di(cyclohexyl-acétyl)-2-[(1R, 2S, 3R) (1, 2, 3, 4-tétrahydroxylbutyl)] 5-[(2'S, 3'R) (2', 3', 4'-trihydroxybutyl)]pyrazine

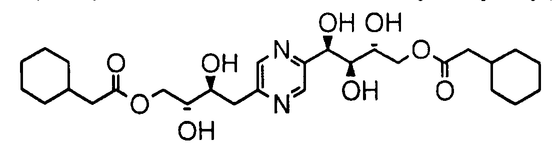

A 350 mg d'acide cyclohexane acétique dans 2 cm³ de dichlorométhane séché sur tamis moléculaire 4Å sont additionnés, goutte à goutte, à une température voisine de 20°C sous atmosphère d'argon, 0,26 cm³ de chlorure d'oxalyle. Le mélange réactionnel est agité à une température voisine de 20°C pendant 1 heure, temps au bout duquel le dégagement gazeux initial a disparu. A 300 mg de 2-[(1R, 2S, 3R) (1, 2, 3, 4-tétrahydroxybutyl)] 5-[(2'S, 3'R) (2', 3', 4'-trihydroxybutyl)] pyrazine en suspension dans 7,5 cm³ de pyridine séchée sur tamis moléculaire 4Å est additionnée, goutte à goutte, à une température voisine de 20°C sous atmosphère d'argon, la solution de chlorure d'acide précédemment préparée. La suspension blanche réactionnelle est agitée 17 heures à une température voisine de 20°C. Le milieu réactionnel est partiellement concentré sous flux d'air à une température voisine de 20°C, puis purifié par chromatographie préparative sur 4 plaques de gel de silice 60F254 Merck (épaisseur = 0,5 mm, 20x20 cm) en éluant par un mélange dichlorométhane-méthanol (90-10 en volumes). Les fractions ne contenant que les produits cherchés sont extraites par un mélange dichlorométhane-méthanol (80-20 en volumes), filtrées sur verre fritté puis concentrées à sec sous pression réduite (0,5 kPa) à une température voisine de 40°C. On obtient ainsi 31,5 mg de 4, 4'-O, O-di(cyclohexyl-acétyl)-2-[(1R, 2S, 3R) (1, 2, 3, 4-tétrahydroxylbutyl)] 5-[(2'S, 3'R) (2', 3', 4'-trihydroxybutyl)] pyrazine sous forme d'un solide blanc.

Le produit obtenu possède les caractéristiques suivantes :
Spectre de R.M.N. ¹H (400 MHz, (CD₃)₂SO d6, δ en ppm) : de 0,85 à 1,80 (mt, 22H en totalité: CH et CH₂ cyclohexyle) ; 2,20 (mt, 4H : OCOCH₂) ; 2,76 et 3,13 (2 dd, respectivement J = 14 et 10 Hz et J = 14 et 3 Hz, 1H chacun : CH₂ 5α) ; de 3,50 à 3,65 (mt, 1H : CH 5γ) ; 3,60 (t large, J = 8,5 Hz, 1H : CH 2β) ; 3,78 (mt, 1H : CH 5β) ; 3,84 (mt, 1H : CH 2γ) ; de 3,95 à 4,10 (mt, 2H : 1H du CH₂O 5δ et 1H du CH₂O 2δ) ; 4,25 (dd, J = 11 et 3 Hz , 1H : l'autre H du CH₂O 5δ) ; 4,30 (dd, J = 12 et 2,5 Hz , 1H : l'autre H CH₂O 2δ) ; 4,62 (d, J = 8,5 Hz, 1H : OH en 2β) ; 4,86 (d, J = 7 Hz, 1H : OH en 5β) ; 4,96 (d large, J = 6,5 Hz, 1H: CH 2α) ; 5,05 (d, J = 6 Hz, 1H : OH en 2γ) ; 5,11 (d, J = 6 Hz, 1H : OH en 5γ) ; 5,41 (d, J = 6,5 Hz, 1H : OH en 2α) ; 8,43 (d, J = 1 Hz, 1H : =CH en 6) ; 8,67 (s large, 1H : =CH en 3).
La 2-[(1R, 2S, 3R) (1, 2, 3, 4-tétrahydroxybutyl)] 5-[(2'S, 3'R) (2', 3', 4'-trihydroxybutyl)] pyrazine peut être préparée à partir de D-glucosamine, en présence d'acide acétique, ou encore par application ou adaptation des méthodes décrites dans Advances in Carbohydrate Chemistry and Biochemistry, Acadelic Press, New York, vol.25, 1970, 311-349.

### Exemple 3

Des souris Swiss albinos pesant entre 22 et 26 g sont laissées à jeun pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des produits selon l'invention.

Les souris ainsi choisies sont réparties en groupes d'au moins 10 animaux. Des groupes distincts reçoivent des doses de 3 à 50 mg/kg de produit dans un véhicule tel que l'eau ou un mélange de méthylcellulose/tween et eau une fois par jour par tubage gastrique . Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les produits selon l'invention présentent un pourcentage d'inhibition de la glycémie supérieur ou égal à 10%.

Un test comparatif selon le protocole décrit ci-dessus a été mené avec le produit de l'exemple 1 selon la présente invention et le produit de référence 2-(1, 2, 3, 4-tétrahydroxybutyl)-5-(2', 3', 4'-trihydroxybutyl)] pyrazine dont l'activité antidiabétique a été décrite dans le demande WO 97/28813 :
Les résultats obtenus après une administration à 3 mg/kg démontrent l'activité relative de 149% du produit de l'exemple 1 par rapport au produit de référence.

### Exemple 4

### EXEMPLE 4A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE 4B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE 4C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Produits de formule générale 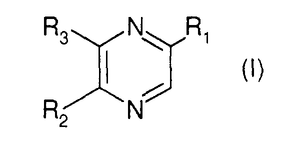 dans laquelle :
R₁ représente les formes stéréoisomères de la chaîne
-(CHOH)₃-CH₂-O-COR (II)
et
soit R₂ représente un atome d'hydrogène et R₃ représente les formes stéréoisomères de la chaîne
-CH₂-(CHOH)₂-CH₂-O-COR (III)
soit R₂ représente les formes stéréoisomères des chaînes
-(CHOH)₃-CH₂-O-COR (II)
ou
-CH₂-(CHOH)₂-CH₂-O-COR (III)
et R₃ représente un atome d'hydrogène
et
R représente un radical -(Alk)ᵢ-(Cycloalk),
pour lequel :
Alk désigne un radical alkyle contenant 1 à 6 atomes de carbone,
Cycloalk désigne un radical cyloalkyle contenant 5 ou 6 atomes de carbone,
i est égal à 0 ou 1;
ainsi que
leurs formes stéréoisomères, et leurs sels avec un acide minéral ou organique.

2. Produits selon la revendication 1 de formules générales : 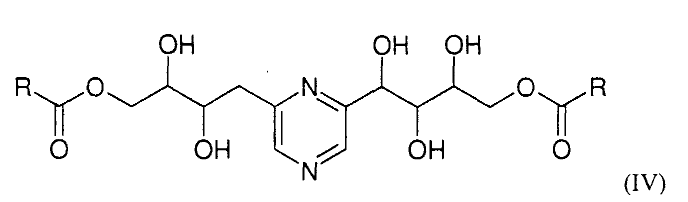 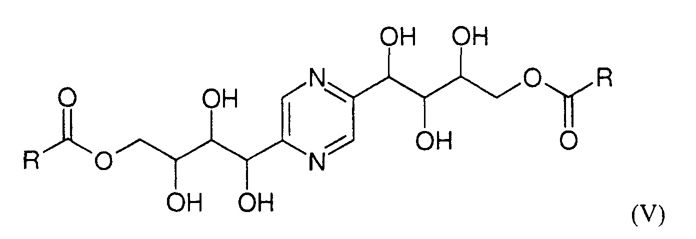 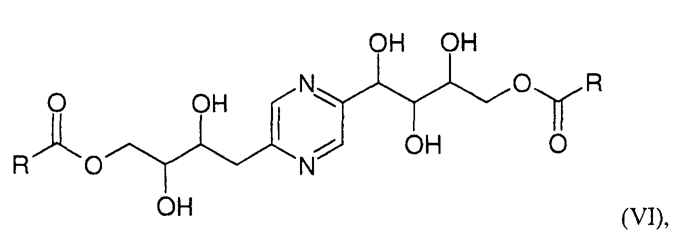 dans lesquelles
R représente un radical -(Alk)ᵢ-(Cycloalk),
pour lequel :
Alk désigne un radical alkyle contenant 1 à 6 atomes de carbone,
Cycloalk désigne un radical cyloalkyle contenant 5 ou 6 atomes de carbone,
i est égal à 0 ou 1;
ainsi que leurs formes stéréoisomères, ou les sels de tels produits avec un acide organique ou minéral.

3. Produits selon l'une quelconque des revendications précédentes de formule générale : 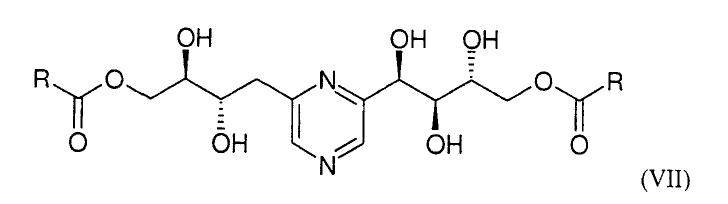 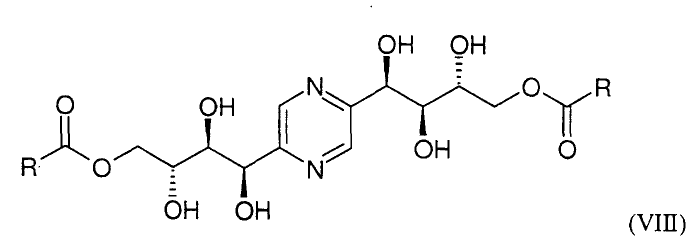 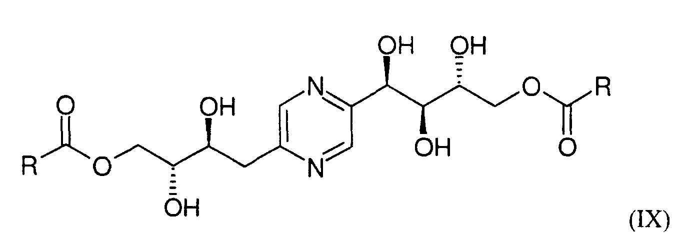 dans lesquelles
R représente un radical
-(Alk)ᵢ-(Cycloalk),
pour lequel :
Alk désigne un radical alkyle contenant 1 à 6 atomes de carbone, Cycloalk désigne un radical cyloalkyle contenant 5 ou 6 atomes de carbone,
i est égal à 0 ou 1;
ainsi que les sels de tels produits avec un acide organique ou minéral.

4. Produits selon l'une quelconque des revendications précédentes de formule générale: 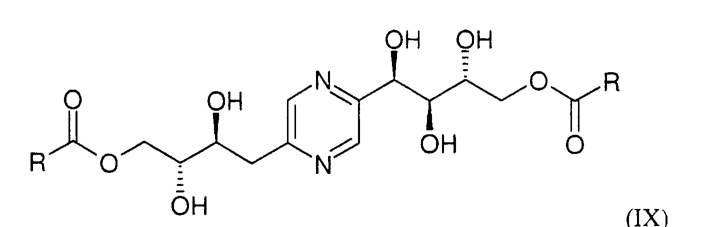 dans laquelle :
R représente un radical
-(Alk)ᵢ-(Cycloalk) ,
pour lequel :
Alk désigne un radical alkyle contenant 1 à 6 atomes de carbone,
Cycloalk désigne un radical cyloalkyle contenant 5 ou 6 atomes de carbone,
i est égal à 0 ou 1;
ainsi que leurs sels avec un acide minéral ou organique.

5. Produits selon l'une quelconque des revendications précédentes pour lesquels :
R représente un radical -(Alk)ᵢ-(Cycloalk),
pour lequel :
Alk désigne le radical méthyle,
Cycloalk désigne un radical cyclohexyle,
i est égal à 0 ou 1;
ainsi que leurs sels avec un acide minéral ou organique.

6. Produits selon l'une quelconque des revendications précédentes choisis individuellement parmi :
4, 4'-O, O-dicyclohexyloyl-2-[(1R, 2S, 3R) (1, 2, 3, 4-tétrahydroxylbutyl)] 5-[(2'S, 3'R) (2', 3', 4'-trihydroxybutyl)] pyrazine
4, 4'-O, O-di(cyclohexyl-acétyl)-2-[(1R, 2S, 3R) (1, 2, 3, 4-tétrahydroxylbutyl)] 5-[(2'S, 3'R) (2', 3', 4'-trihydroxybutyl)] pyrazine
ainsi que leurs sels avec un acide minéral ou organique.

7. Le produit selon l'une quelconque des revendications précédentes qui est le 4, 4'-O, O-dicyclohexyloyl-2-[(1R, 2S, 3R) (1, 2, 3, 4-tétrahydroxylbutyl)] 5-[(2'S, 3'R) (2', 3', 4'-trihydroxybutyl)] pyrazine ainsi que ses sels avec un acide minéral ou organique.

8. Procédé de préparation des produits selon l'une quelconque des revendications précédentes, **caractérisé en ce que** l'on procède à partir des produits de formule générale : 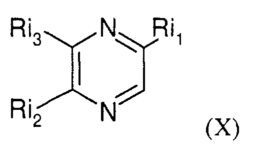 dans laquelle :
Ri₁ représente les formes stéréoisomères de la chaîne
-(CHOH)₃-CH₂OH (XI)
et
soit Ri₂ représente un atome d'hydrogène et Ri₃ représente les formes stéréoisomères de la chaîne
-CH₂-(CHOH)₂-CH₂OH (XII)
soit Ri₂ représente les formes stéréoisomères des chaînes
-(CHOH)₃-CH₂OH (XI)
ou
-CH₂-(CHOH)₂-CH₂OH (XII)
et Ri₃ représente un atome d'hydrogène,
par action d'halogénure d'acyle correspondant, de formule R-COX, dans laquelle R est défini comme dans les revendications 1 à 7 et X représente un atome d'halogène.

9. Procédé selon la revendication 8 **caractérisé en ce que** la réaction est effectuée en présence de pyridine, à des températures comprises entre 0 et 40°C.

10. Médicaments **caractérisés en ce qu'**ils contiennent en tant que principe actif un ou plusieurs produits selon l'une quelconque des revendications 1 à 7 et un ou plusieurs excipients.

11. Utilisation des produits selon l'une quelconque des revendications 1 à 7 pour la préparation d'un médicament pour la prévention et/ou traitement du diabète et des complications du diabète.

## Patentansprüche

1. Produkt der allgemeinen Formel 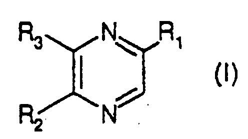 worin
R₁ die stereoisomeren Formen der Kette
-(CHOH)₃-CH₂-O-COR (II)
darstellt und
entweder R₂ ein Wasserstoffatom darstellt und R₃ die stereoisomeren Formen der kette
-CH₂-(CHOH)₂-CH₂-O-COR (III)
darstellt oder
R₂ die stereoisomeren Formen der Ketten
-(CHOH)₃-CH₃-O-COR (II)
oder
-CH₂-(CHOH)₂-CH₂-O-COR (III)
darstellt und R₃ ein Wasserstoffatom darstellt und
R eine Gruppe -(Alk)ᵢ-(Cycloalk) darstellt, worin Alk eine Alkyl-Gruppe, die 1 bis 6 Kohlenstoffatome enthält, bedeutet; Cycloalk eine Cycloalkyl-Gruppe, die 5 oder 6 Kohlenstoffatome enthält, bedeutet;
i gleich 0 oder 1 ist;
sowie deren stereoisomeren Formen und ihre Salze mit einer Mineralsäure oder einer organischen Säure.

2. Produkte nach Anspruch 1 der allgemeinen Formeln: 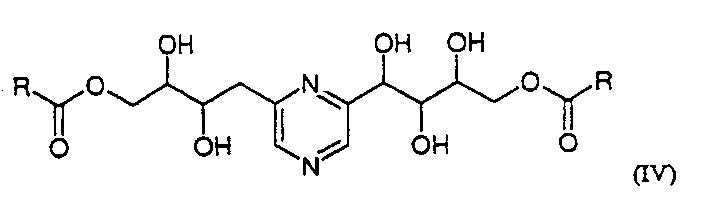 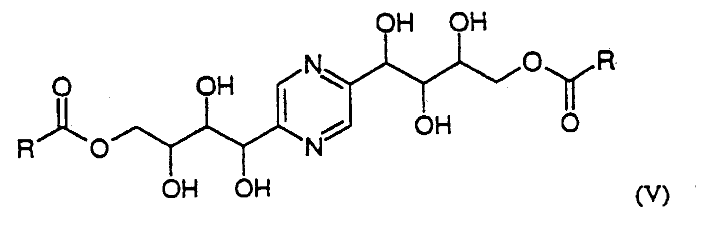 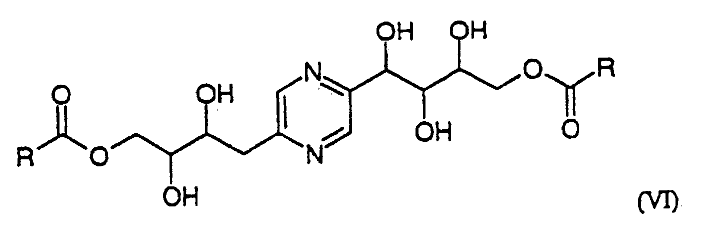 worin
R eine Gruppe -(Alk)ᵢ-(Cycloalk) darstellt, worin Alk eine Alkyl-Gruppe, die 1 bis 6 Kohlenstoffatome enthält, bedeutet;
Cycloalk eine Cycloalkyl-Gruppe, die 5 oder 6 Kohlenstoffatome enthält, bedeutet;
i gleich 0 oder 1 ist;
sowie deren stereoisomeren Formen oder die Salze mit einer Mineralsäure oder einer organischen Säure.

3. Produkte nach einem der vorangehenden Ansprüche mit der allgemeinen Formel: 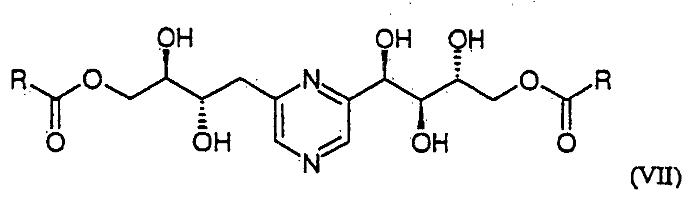 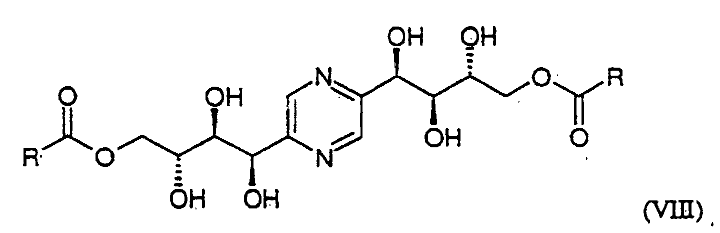 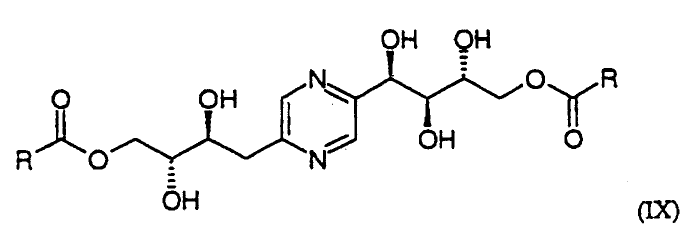 in denen
R eine Gruppe -(Alk)ᵢ-(Cycloalk) darstellt, worin:
Alk eine Alkyl-Gruppe, die 1 bis 6 Kohlenstoffatome enthält, bedeutet,
Cycloalk eine Cycloalkyl-Gruppe, die 5 oder 6 Kohlenstoffatome enthält, bedeutet,
i gleich 0 oder 1 ist;
sowie die Salze der Produkte mit einer organischen Säure oder einer Mineralsäure.

4. Produkte nach einem der vorangehenden Ansprüche mit der allgemeinen Formel: 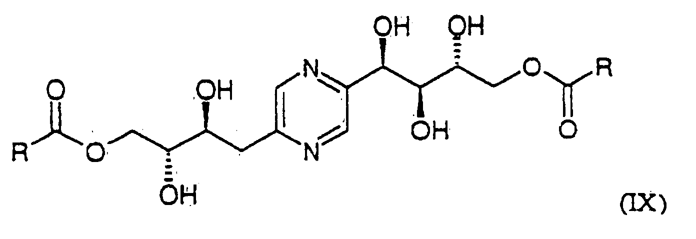 worin
R eine Gruppe -(Alk)ᵢ-(Cycloalk) darstellt, worin Alk eine Alkyl-Gruppe, die 1 bis 6 Kohlenstoffatome enthält bedeutet;
Cycloalk eine Cycloalkyl-Gruppe, die 5 oder 6 Kohlenstoffatome enthält, bedeutet;
i gleich 0 oder 1 ist;
sowie deren Salze mit einer Mineralsäure oder einer organischen Säure.

5. Produkte nach einem der vorangehenden Ansprüche, für die:
R eine Gruppe -(Alk)ᵢ-(Cycloalk) bedeutet, worin Alk die Methyl-Gruppe bedeutet,
Cycloalk eine Cyclohexyl-Gruppe bedeutet;
i 0 oder 1 ist;
sowie ihre Salze mit einer Mineralsäure oder organischen Säure.

6. Produkte nach einem der vorangehenden Ansprüche, individuell ausgewählt unter:
4,4'-O,O-Dicyclohexyloyl-2-[(1R,2S,3R)(1,2,3,4-tetrahydroxylbutyl)]-5-[(2'S,3'R)(2',3',4'-trihydroxybutyl)]pyrazin,
4,4'-O,O-Dicyclohexyl-acetyl)-2-[(1R,2S,3R)-(1,2,3,4-tetrahydroxylbutyl)1-5-[(2'S,3'R) (2',3',4'trihydroxybutyl)]pyrazin
sowie ihren Salzen mit einer Mineralsäure oder einer organischen Säure.

7. Produkt nach einem der vorangehenden Ansprüche, das 4,4'-O,O-Dicyclohexyloyl-2-[(1R,2S,3R)(1,2,3,4-tetrahydroxylbutyl)]-5-[(2'S,3'R)(2',3',4'-trihydroxybutyl)]pyrazin sowie seine Salze mit einer Mineralsäure oder einer organischen Säure ist.

8. Verfahren zur Herstellung der Produkte nach einem der vorangehenden Ansprüche, **dadurch gekennzeichnet, daß** man Produkte der allgemeinen Formel: 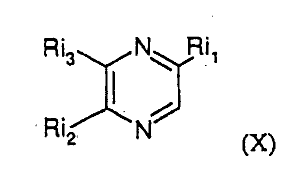 worin
Ri₁ die stereoisomeren Formen der Kette
-(CHOH)₃-CH₂OH (XI)
darstellt und
entweder Ri₂ ein Wasserstoffatom darstellt und Ri₃ die stereoisomeren Formen der Kette
-CH₂-(CHOH)₂-CH₂OH (XII)
darstellt oder Ri₂ die stereoisomeren Formen der Ketten
-(CHOH)₃-CH₂OH (XI)
oder
-CH₂-(CHOH)₂-CH₂OH (XII)
darstellt und Ri₃ ein Wasserstoffatom darstellt,
mit einem entsprechenden Acylhalogenid der Formel R-COX, worin R wie in den Ansprüchen 1 bis 7 definiert ist und X ein Halogenatom darstellt, umsetzt.

9. Verfahren nach Anspruch 8, **dadurch gekennzeichnet, daß** die Reaktion in Gegenwart von Pyridin bei Temperaturen zwischen 0 und 40°C durchgeführt wird.

10. Arzneimittel, **dadurch gekennzeichnet, daß** sie als Wirkstoff ein Produkt oder mehrere Produkte nach einem der Ansprüche 1 bis 7 und ein Exzipienz oder mehrere Exzipienzien enthalten.

11. Verwendung der Produkte nach einem der Ansprüche 1 bis 7 zur Herstellung eines Arzneimittels zur Prävention und/oder Behandlung von Diabetes und Komplikationen von Diabetes.

## Claims

1. Products of general formula 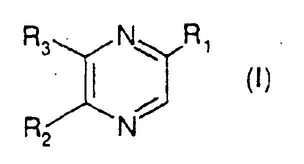 in which:
R₁ represents the stereoisomeric forms of the chain
-(CHOH)₃-CH₂-O-COR (II)
and
either R₂ represents a hydrogen atom and R₃ represents the stereoisomeric forms of the chain
-CH₂-(CHOH)₂-CH₂-O-COR (III)
or R₂ represents the stereoisomeric forms of the chains
- (CHOH)₃-CH₂-O-COR (II)
or
-CH₂-(CHOH)₂-CH₂-O-COR (III)
and R₃ represents a hydrogen atom
and
R represents an-(Alk)ᵢ-(Cycloalk) radical,
for which:
Alk denotes an alkyl radical containing from 1 to 6 carbon atoms,
Cycloalk denotes a cycloalkyl radical containing 5 or 6 carbon atoms,
i is equal to 0 or 1;
and
their stereoisomeric forms and their salts with an inorganic or organic acid.

2. Products according to Claim 1 of general formulae: 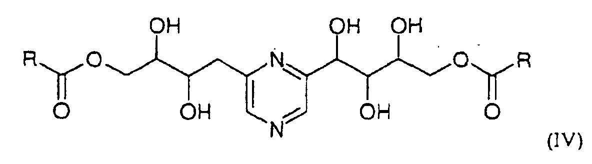 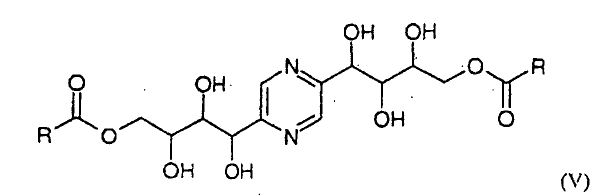 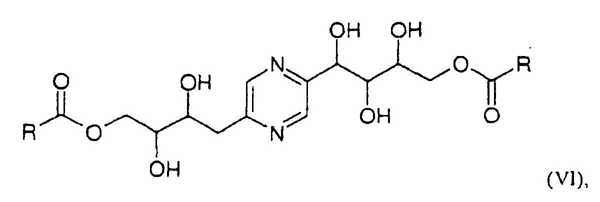 in which
R i represents an -(Alk)ᵢ-(Cycloalk) radical,
for which:
Alk denotes an alkyl radical containing from 1 to 6 carbon atoms,
Cycloalk denotes a cycloalkyl radical containing 5 or 6 carbon atoms,
i is equal to 0 or 1;
and their stereoisomeric forms or the salts of such products with an organic or inorganic acid.

3. Products according to either one of the preceding claims of general formulae: 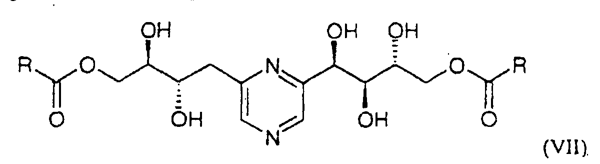 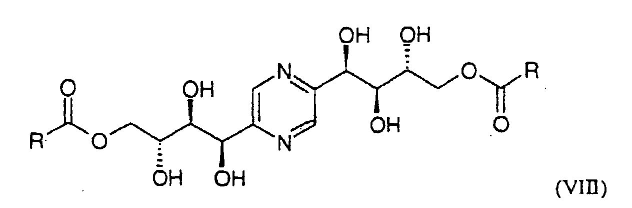 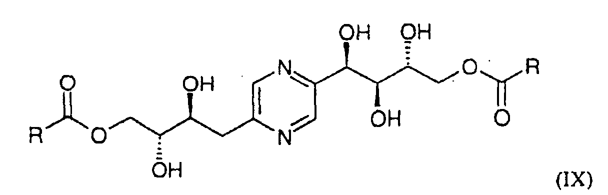 in which
R represents an-(Alk)ᵢ-(Cycloalk) radical,
for which:
Alk denotes an alkyl radical containing from 1 to 6 carbon atoms,
Cycloalk denotes a cycloalkyl radical containing 5 or 6 carbon atoms,
i is equal to 0 or 1;
and the salts of such products with an organic or inorganic acid.

4. Products according to any one of the preceding claims of general formula: 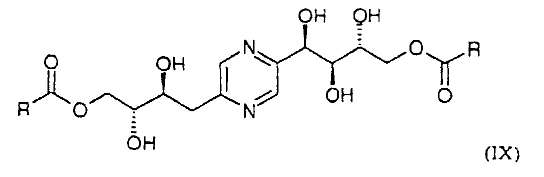 in which:
R represents an-(Alk)ᵢ-(Cycloalk) radical,
for which:
Alk denotes an alkyl radical containing from 1 to 6 carbon atoms,
Cycloalk denotes a cycloalkyl radical containing 5 or 6 carbon atoms,
i is equal to 0 or 1;
and their salts with an organic or inorganic acid.

5. Products according to any one of the preceding claims for which:
R represents an-(Alk)ᵢ-(Cycloalk) radical,
for which:
Alk denotes the methyl radical,
Cycloalk denotes a cyclohexyl radical,
i is equal to 0 or 1;
and their salts with an organic or inorganic acid.

6. Products according to any one of the preceding claims chosen individually from:
4,4'-O,O-dicyclohexylcarbonyl-2-[(1R,2S,3R)(1,2,3,4-tetrahydroxybutyl)]-5-[(2'S,3'R)(2',3',4'-trihydroxybutyl)]pyrazine
4,4'-O,O-di(cyclohexylacetyl)-2-[(1R,2S,3R)(1,2,3,4-tetrahydroxybutyl)]-5-[(2'S,3'R)-(2',3',4'-trihydroxybutyl)]pyrazine
and their salts with an inorganic or organic acid.

7. Product according to any one of the preceding claims which is 4,4'-O,O-Dicyclohexylcarbonyl-2-[(1R,2S,3R)(1,2,3,4-tetrahydroxybutyl)]-5-[(2'S,3'R)(2',3',4'-trihydroxybutyl)]pyrazine and its salts with an inorganic or organic acid.

8. Process for the preparation of the products according to any one of the preceding claims, **characterized in that** the preparation is carried out from the products of general formula: 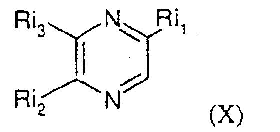 in which:
Ri₁ represents the stereoisomeric forms of the chain
-(CHOH)₃-CH₂OH (XI)
and
Ri₂ represents a hydrogen atom and Ri₃ represents the stereoisomeric forms of the chain
-CH₂-(CHOH)₂-CH₂OH (XII)
or Ri₂ represents the stereoisomeric forms of the chains
-(CHOH)₃-CH₂OH (XI)
or
-CH₂-(CHOH)₂-CH₂OH (XII)
and Ri₃ represents a hydrogen atom,
by reaction with a corresponding acyl halide of formula R-COX, in which R is defined as in Claims 1 to 7 and X represents a halogen atom.

9. Process according to Claim 8, **characterized in that** the reaction is carried out in the presence of pyridine at temperatures of between 0 and 40°C.

10. Medicaments, **characterized in that** they comprise, as active principle, one or more products according to any one of Claims 1 to 7 and one or more excipients.

11. Use of the products according to any one of Claims 1 to 7 in the preparation of a medicament for the prevention and/or treatment of diabetes and complications of diabetes.
